# EUROPEAN PATENT SPECIFICATION

(11) **EP 1 964 614 B1**
(45) Date of publication and mention of the grant of the patent: **18.01.2012**
(21) Application number: 06834960.4
(22) Date of filing: 19.12.2006
(51) Int. Cl.: B05B 5/025, B05B 5/16

(54) **ELECTROSTATIC ATOMIZER AND FOOD PRESERVING CABINET WITH ELECTROSTATIC ATOMIZER**
ELEKTROSTATISCHER ZERSTÄUBER UND SCHRANK ZUM AUFBEWAHREN VON LEBENSMITTELN MIT ELEKTROSTATISCHEM ZERSTÄUBER
ATOMISEUR ELECTROSTATIQUE ET CHAMBRE DE CONSERVATION D' ALIMENTS A ATOMISEUR ELECTROSTATIQUE

(30) Priority: 22.12.2005 JP 2005371055
(43) Date of publication of application: 03.09.2008
(73) Proprietor: Panasonic Electric Works Co., Ltd., Kadoma-shi Osaka (JP)
(72) Inventor: SUDA, Hiroshi c/o Panasonic Electric Works Co., Ltd., Kadoma-shi, Osaka 571-8686 (JP); NAKADA, Takayuki c/o Panasonic Electric Works Co., Ltd., Kadoma-shi, Osaka 571-8686 (JP); MACHI, Masaharu c/o Panasonic Electric Works Co., Ltd., Kadoma-shi, Osaka 571-8686 (JP)
(74) Representative: Appelt, Christian W.
(86) International application number: PCT/JP2006/325250
(87) International publication number: WO 2007/072811

(56) References cited:
- WO-A-2004/078244
- JP-A- 09 010 632
- JP-A- 11 094 446
- JP-A- 2000 304 311
- JP-A- 2004 275 923
- JP-A- 2005 164 139
- JP-A- 2005 164 139
- JP-A- 2005 265 313
- JP-U- 04 057 261
- US-B1- 6 397 838
- US-B1- 6 679 441
- CLOUPEAU M ET AL: "ELECTROSTATIC SPRAYING OF LIQUIDS IN CONE-JET MODE" JOURNAL OF ELECTROSTATICS, ELSEVIER SCIENCE PUBLISHERS B.V. AMSTERDAM, NL, vol. 22, no. 2, 1 July 1989 (1989-07-01), pages 135-159, XP000039067 ISSN: 0304-3886

## Description

### TECHNICAL FIELD

This innovation relates to an electrostatically atomizing device which has a function of an efficient humidification as well as functions of a sterilization, a deodorization, and a efficient decomposition of a harmful substance, and a food container which incorporates the electrostatically atomizing device.

### BACKGROUND ART

Japanese patent application no.2005-131549A discloses an electrostatically atomizing device which generates a mist of charged minute water particles by a method to atomize water electrically. The electrostatically atomizing device causes the Rayleigh breakup to the water of the emitter electrode, atomizes the water by the Rayleigh breakup, and then generates the mist of the charged minute water particles of nanometer order. The mist of the charged minute water particles includes radicals, is able to float in air over an extended time period, and is able to spread into the air in large quantity. The mist of the charged minute water particles adheres to substances of space where the mist of the charged minute water particles is discharged, penetrates the substances, and subsequently sterilizes and deodorizes the substances. The mist of the charged minute water particles is able to humidify the space. However, the mist of the charged minute water particles has diameters of the nanometer order. Even after the electrostatically atomizing device generates a large amount of the mist of the charged minute water particles of the nanometer order, the electrostatically atomizing device discharges only a little water. The electrostatically atomizing device is not able to sufficiently humidify the space. In the case of using the electrostatically atomizing device in the space where the humidification is required, the electrostatically atomizing device is generally used with a traditional humidifier which generates water vapor.

From US 6,679,441 B1 an electrohydrodynamic spraying means enabling spraying of liquids with high surface tension such as water, especially for surface coating or deposition, is known.

Furthermore, document JP 2005-164139 discloses a humidifier to be used independently or incorporated in other equipment, such as e.g. in air conditioner, capable of spraying fine water droplets in a desired direction without using a fan, a variable guide or louver.

### DISCLOSURE OF THE INVENTION

In view of the above problem, the present invention is achieved to provide an electrostatically atomizing device which is capable of humidifying the space as well as decomposing the harmful substances, sterilizing the substances, and deodorizing the substances in the space, and to provide a food container which incorporates the electrostatically atomizing device.

This object is solved by an electrostatic atomizing device according to claim 1, claims 2 to 7 relate to specific advantages of an inventive electrostatically atomizing device according to claim 1.

The electrostatically atomizing device in accordance with the present invention includes a liquid carrier formed at its one end with an emitter electrode, an opposed electrode disposed in an opposed relation to the emitter electrode, a liquid supplying means for supplying a liquid to the liquid carrier, and a high voltage source configured to apply a high voltage between the emitter electrode and the opposed electrode to electrically-charge the liquid fed to a tip of the emitter electrode for discharging a mist of charged minute water particles from the tip of the emitter electrode. The liquid supplying means includes a pressurizing means configured to apply a pressure to the liquid on the emitter electrode for discharging from the tip of the emitter electrode the mist of the charged minute water particles of a size in a wide range from a nanometer order of 3nm to 100nm to a micron order of 0.1 µm to 10µm. The high voltage source applies the high voltage at the tip of the emitter electrode. The high voltage of the tip of the emitter electrode forms a Taylor cone which is formed by the surface tension and at the tip of the emitter electrode. The high voltage of the tip of the emitter electrode causes a concentration of the electric charge at a tip of the Taylor cone and causes the water to break up. As a result, the mist of the charged minute water particles of the nanometer order is mainly generated and is discharged. In addition, the Taylor cone which is formed by the surface tension receives the pressure by the pressurizing means. The pressure upsets the balance of the form of the Taylor cone which is formed by the surface tension. For this reason, the liquid of some parts other than the tip of the Taylor cone also breaks up. And then, the mist of the charged minute water particles is generated from the some parts other than the tip of the Taylor cone. The electrically-charge is hardly concentrated at the tip of the Taylor cone. As a result, the liquid has little energy for breaking up. Consequently, the mist of the charged minute water particles of micron order is generated from the some parts other than the tip of the Taylor cone. In this case, the electrostatically atomizing device is able to humidify the space by the mist of the charged minute water particles of micron order which is discharged with the mist of the charged minute water particles of nanometer order as well as is able to decompose the harmful substances in the space, and is able to sterilize and deodorize the substances in the space by the mist of the charged minute water particles of nanometer order.

The liquid carrier is of a tubular configuration composed of a main tube and a capillary tube which extends from the main tube and which defines the emitter electrode. The main tube has an inside diameter sufficiently larger than that of the capillary tube so as to cause no capillary action. The main tube is provided at its rear end with a pressurizing tank which defines the liquid supplying means such that the liquid stored in the pressurizing tank applies the pressure to the liquid at the tip of the capillary tube. In this way, the emitter electrode of the electrostatically atomizing device generates the mist of the charged minute water particles of nanometer order and the mist of the charged minute water particles of micron order. With this arrangement, the pressurizing tank is able to apply a suitable pressure to the liquid which is supplied to the emitter electrode by the capillary action and which is held at the tip of the emitter electrode. Consequently, the emitter electrode of the electrostatically atomizing device is able to generate the mist of the charged minute water particles on nanometer order and micron order.

A replenishing tank is coupled to the pressurizing tank, and a level sensor is configured to detect a liquid level of the liquid in the pressurizing tank, and a replenishing means is configured to add the liquid from replenishing tank to the pressurizing tank in order to keep the liquid level detected by the level sensor at a constant level. In this case, the pressuring tank is able to apply a constant water head pressure to the liquid at the tip of the emitter electrode. As a result, the emitter electrode of the electrostatically atomizing device is able to generate the mist of the charged minute water particles of nanometer order and micron order which have stable particle size distribution.

Furthermore, it is also preferable that the pressurizing tank includes a piston for pressurizing the liquid. In this case, the pressuring tank is able to apply the pressure to the liquid by using the piston without using the replenishing tank. Consequently, the emitter electrode of the electrostatically atomizing device is able to generate the mist of the charged minute water particles of stable particle size distribution.

It is preferable that the mist of the charged minute water particles of the size in the nanometer order exhibits a particle size distribution having a peak at 3nm to 50nm, and the mist of the charged minute particles of the size in the micron order exhibits a particle size distribution having a peak at 0.5µm to 1.5 µm. The mist of the charged minute water particles of nanometer order and micron order having previously described particle size distributions are able to sufficiently humidify the space as well as is able to decompose the harmful substances, sterilize the substances, and deodorize the substances in the space. Especially, in the case of using the electrostatically atomizing device of the present invention for a vegetable compartment of the container, the mist of the charged minute water particle of micron order having the above mentioned particle size distribution penetrates an inside of vegetables through the vegetable stomata of 100-200 µm long and 10µm wide, supplies the water to the vegetables, and keeps the vegetable fresh. In addition, the mist of the charged minute water particles of nanometer order decomposes the harmful substances such as agrichemical which adheres to the vegetables, sterilizes the vegetable, and deodorizes the vegetable. Furthermore, the mist of the charged minute water particles of micron order which has the peak of the particle size distribution of 0.5 µm to 1.5 µm is able to supply the water to various vegetables efficiently. The above mentioned particle size distribution is able to adjust by varying the pressure. Consequently, it is preferable that a pressure regulating means is provided to regulate the pressure applied to the liquid by the pressurizing means.

In addition, it is preferable that the liquid carrier includes a filter configured to entrap mineral components contained in the liquid. In the case of using tap water as the liquid, the filter entraps the mineral components such as Ca and Mg, and prevents the mineral components from depositing to the emitter electrode. Consequently, the emitter electrode is able to electrostatically atomize.

Preferably, the electrostatically atomizing device is arranged in the food container. The previous described food container is able to humidify the food of the inside of the food container and is able to keep the foods fresh by the mist of the charged minute water particles of micron order as well as is able to decompose the harmful substances, sterilize foods, and deodorizing the foods in the food container by the mist of the charged minute water particles of nanometer order.

### BRIEF DESCRIPTION OF THE DRAWING

FIG.1 shows a schematic view of an electrostatically atomizing device in accordance with an embodiment of the present invention,
FIG.2 shows a perspective view of the above electrostatically atomizing device,
FIG.3 shows a perspective view of the above electrostatically atomizing device in the state that the cover is removed,
FIG.4 shows a schematic view of an electrostatically atomizing device in accordance with another embodiment of the present invention,
FIG.5 shows a schematic view of a food container provided with the electrostatically atomizing device in accordance with the present invention, and
FIG.6 shows the graph explaining a preservation of a freshness of leafy vegetables in using the electrostatically atomizing device in accordance with the present invention.

### BEST MODE FOR CARRYING OUT THE INVENTION

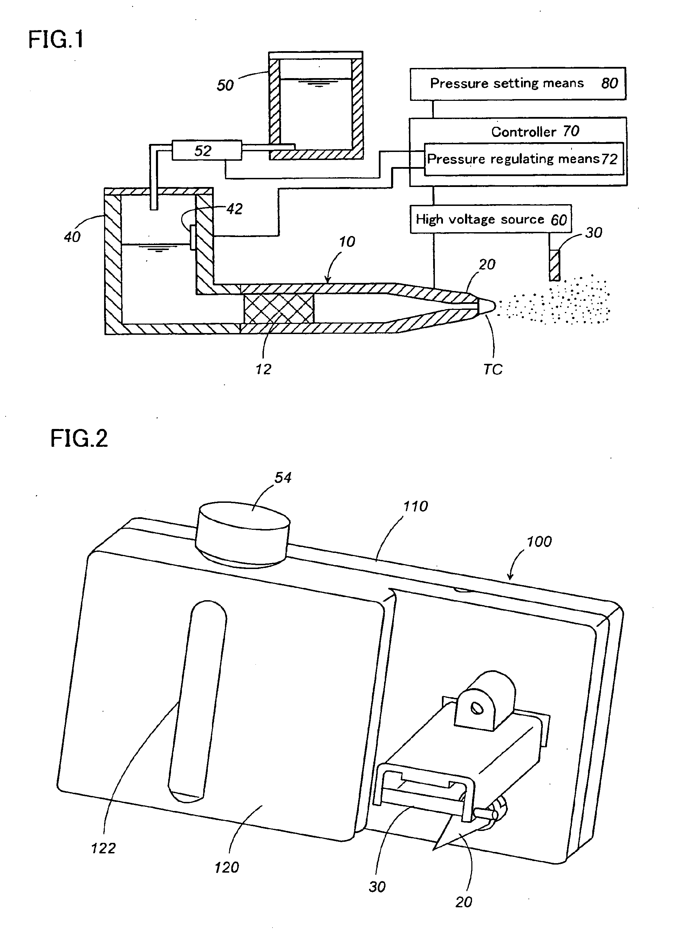

Now a reference is made to the attached drawings to explain an electrostatically atomizing device in accordance with one embodiment of the present invention. As shown in FIG.1, the electrostatically atomizing device includes a liquid carrier 10 which is formed at its one end with an emitter electrode 20, an opposed electrode 30, a high voltage source 60, and a controller 70. The opposed electrode 30 is disposed in an opposed relation to the emitter electrode 20. The high voltage source 60 is provided in order to apply a high voltage between the emitter electrode 20 and the opposed electrode 30. The controller 70 is configured to control a high voltage value of the applied high voltage. The liquid carrier 10 has a rear end which is connected to a pressuring tank 40. The pressuring tank 40 supplies a liquid which is stored in the pressuring tank such as water to the one end of the emitter electrode through the liquid carrier 10. The pressuring tank 40 defines the liquid supplying means for supplying the liquid to the liquid carrier 10 and the pressurizing means for applying the pressure to the liquid. The electrostatically atomizing device of this embodiment is explained by using the water as the liquid. However, the electrostatically atomizing device of this invention also is able to use the liquid other than the water.

The water is supplied to the emitter electrode and forms a ball of the water at the tip of the emitter electrode by a surface tension. The high voltage source applies the high voltage such as -8kV to the emitter electrode 20 and generates the high voltage electric field between the opposed electrode 30 and the discharge end of the emitter electrode. The high voltage electric field electrically-charges the water by the static electrical charge and causes the emitter electrode 20 to discharge the mist of the charged minute water particles . The high voltage which is applied between the emitter electrode 20 and the opposed electrode 30 generates the Coulomb force between the opposed electrode 30 and the water which is held at the one end of the emitter electrode, thereby forming the Taylor cone TC which protrudes from the surface of the ball of the water. Then, the electric charge concentrates to the tip of the Taylor cone TC. The electric field intensity of the tip of the Taylor cone becomes larger. As the electric field intensity of the tip of the Taylor cone becomes larger, the Coulomb force of the tip of the Taylor cone becomes larger, thereby developing the large Taylor cone TC. When the Coulomb force becomes larger than the surface tension of the water W, the Taylor cone repeats the breakup (Rayleigh breakup). As a result, a large amount of the mist of the charged minute water particles of nanometer order is generated. The mist of the charged minute water particles of nanometer order is carried by an airflow which is caused by an ion wind which flows from the emitter electrode 20 to the opposed electrode 30, and is discharged through the opposed electrode 30.

The pressuring tank 40 is supplied with the water from a replenishing tank 50 by a pump 52. A water level of the pressuring tank 40 is always controlled to a same water level. The pressuring tank which is kept to keep a same water level applies a constant water head pressure to the one end of the emitter electrode 20. For this reason, the pressuring tank 40 is provided with a level sensor 42. The pump 52 is controlled by a pressure regulating means 72, thereby keeping the water level which is sensed by the level sensor 42 to the constant water level. The pressure regulating means 72 constitutes the controller 70 and controls the pump 52 to generate a pressure value, namely the water head pressure which is configured by a pressure setting means 80.

The liquid carrier 10 is a the tubular configuration. The liquid carrier 10 is formed at its one end with the emitter electrode 20 which is formed into a capillary tube. The some parts of the liquid carrier 10 across the pressuring tank 40 and the emitter electrode 20 is formed to have an inner diameter which does not take place the capillary action. Thus, the water head pressure is applied to the ball of the water which is formed at the emitter electrode 20. The inner diameter of the liquid carrier 10 gradually becomes smaller toward its one end which is the capillary tube. The water is supplied to the tip of the emitter electrode through the capillary tube and forms the ball of the water at the tip of the emitter electrode by the surface tension. The water head pressure is controlled not to prevent the surface tension from forming the ball of the water. The water head pressure is applied to the Taylor cone TC which is formed by the high voltage.

The water head pressure is applied to the Taylor cone TC which has the form kept by the surface tension. By applying the high voltage, the some parts other than the tip of the surface of the Taylor cone where the electric-charge is concentrated is also breakup. The amount of the electric-charge of the some parts other than the tip of the Taylor cone is lessen than the amount of the electric-charge at the tip of the Taylor cone. The some parts other than the tip of the Taylor cone have a little energy to break up the water. As a result, it is considered that the mist of the charged minute water particles of micron order is mainly generated. Consequently, by applying the high voltage to the water which is held at the tip of the emitter electrode 20 and which receives the pressure, the mist of the charged minute water particles of nanometer order is generated from the tip of the Taylor cone TC. And, the mist of the charged minute water particles of micron order is generated from the some parts other than the tip of the Taylor cone TC. The mists of the charged minute water particles of nanometer order and micron order are discharged to the space in the diffused state. The emitter electrode 20 continues being supplied with the water by the pressure, and generates the mist of the charged minute water particles continuously.

The mist of the charged minute water particles of nanometer order includes radicals. The radicals decomposes the harmful substances, sterilizes the substances in the space, and deodorizes the substances in the space. The mist of the charged minute water particles of micron order spreads into the space and humidifies the space.

The pressure regulating means 72 varies the pressure for applying the emitter electrode 20, thereby adjusting the particle size distribution and the generation amount of the mists of the charged minute water particles of nanometer order and micron order. Namely, according to the pressure which is configured by the pressure setting means 80, the particle size distribution and a ratio of the generation amount is able to select. The emitter electrode 20 is able to generate a mixture mist which includes optimum quantities of the mists of the charged minute water particles of nanometer order and micron order as usage.

In this application, the nanometer order is defined in a range which is equal to or more than 3nm and is equal to or less than 100nm. The micron order is defined in a range which exceeds 0.1 µm and is equal to or less than 10 µm.

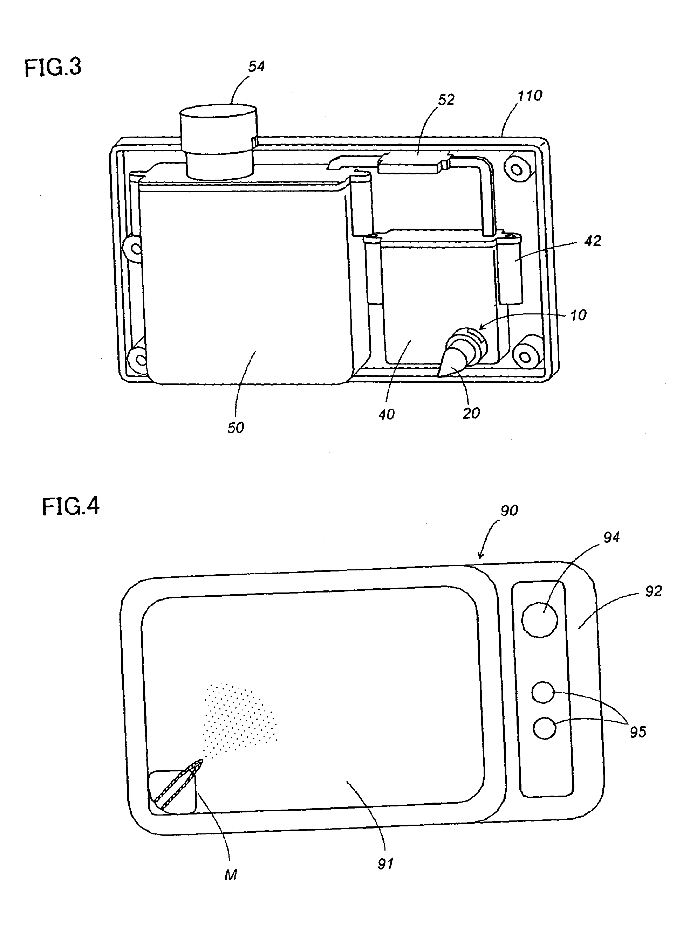

The above mentioned parts which constitute the electrostatically atomizing device are incorporated into a housing 100 which is shown in FIG. 2 and FIG.3. The housing 100 includes a base 110 and a cover 120 which covers the base 110. The base 110 holds the liquid carrier 10 which is integrated with the pressuring tank 40, the replenishing tank 50, and the pump 52. The cover 120 holds the opposed electrode 30. An outside surface of the housing 100 is provided with the emitter electrode 20 and the opposed electrode 30. The housing 100 incorporates electrical parts which constitutes the high voltage source 60, the controller 70, and the pressure setting means 80. The cover 120 is provided with a window 122. The window 122 is provided in order to check the water level of the replenishing tank 50 which is made of a transparent material. The replenishing tank 50 is provided with the cap 54 and is supplied with the water according to need.

In the electrostatically atomizing device in accordance with the present invention, the liquid carrier 10 is provided with the filter 12 which entraps mineral components such as Ca and Mg. In this way, the filter 12 prevents the mineral components from depositing to the emitter electrode 20 when the tap water is used to the electrostatically atomizing device.

FIG.4 shows the electrostatically atomizing device which is incorporated into the food container 90 which stores the foods such as the vegetables. The food container 90 is able to decompose the harmful substances such as agrichemicals, sterilize the foods, and deodorize the foods by the mist of the charged minute water particles of nanometer order. The food container 90 is able to maintain the suitable humidity in an inside space of the food container 90 by the mist of the charged minute water particles of micron order. Especially in the case of storing the vegetables in the food container, a large amount of the charged minute water particles of micron order are supplied to the vegetables tissue through the stomata of the vegetables. In this way, the food container is able to keep the vegetables fresh.

The food container 90 is provided with a temperature regulator 92 for keeping a predetermined temperature. An outside surface of the temperature regulator 92 is provided with a power button 94 and a temperature regulating button 95. The electrostatically atomizing device M is operated by the power button 94 and discharges the mists of the charged minute water particles of nanometer order and micron order to a container 91.

It is known that the leafy vegetables are not able to keep its freshness by only supplying the water to a surface of a leaf but is able to keep its freshness by supplying the water to the tissues of the leaf through the stomata of the leaf. The stomata of the leaf of the leafy vegetables are about 100-200µm long and about 10µm wide. Because the mist of the charged minute water particles of nanometer order has extremely small particle diameter, the mist of the charged minute water particles of nanometer order penetrates into the tissues of the leaf through the stomata of the leaf, but is not able to supply the necessary water to the leafy vegetables for keeping the leafy vegetables fresh. However, the mist of the charged minute water particles of micron order has large amount of the water than that of nanometer order. The charged minute water particles of micron order is able to penetrate to the tissue of the leaf through the stomata of the leaf, is able to supply a sufficient amount of the water to the tissue of the leafy vegetables, and is able to keep the leafy vegetables fresh. For this reason, the electrostatically atomizing device which is incorporated into the food container is adjusted to the suitable pressure and the suitable applied voltage for generating the mist of the charged minute water particles of micron order which has a peak of the particle size distribution in a range which is equal to or less than 10µm (preferably, is equal to or more than 0.5µm and is equal to or less than 1.5µm).

In addition, the mist of the charged minute water particles of nanometer order is able to penetrate into the tissue of the leafy vegetables through the stomata, is able to decompose the harmful substances such as the agrichemicals which penetrates to an inside of the tissues of the leafy vegetables, is able to sterilize and deodorize the inside of the tissues of the leafy vegetables as well as is able to decompose the harmful substances which adhere to the leafy vegetables, is able to sterilize the leafy vegetables, and is able to deodorize the leafy vegetables. In this case, the pressure and the applied voltage is adjusted for generating the mist of the charged minute water particles of nanometer order which has the particle size distribution in a range which is equal to or more than 3nm and is equal to or less than 50nm.

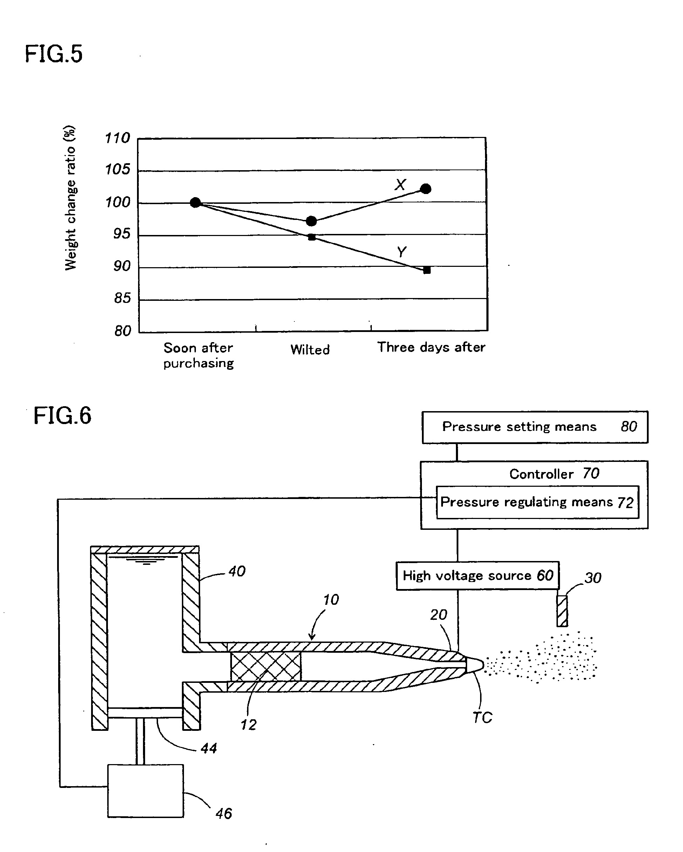

FIG.5 shows a freshness keeping effect (activity effect) of the leafy vegetables in the food container which incorporates the above mentioned electrostatically atomizing device. In this experience, a stick of wilted celery was prepared as a sample and was put in the container 91 which was a 30liter in volume. The inside of the container 91 was illuminated into a blue LEDs as a dummy sunlight, was kept at 5 degrees Celsius, and was kept at a humidity of 99%. The electrostatically atomizing device M discharged the mist of the charged minute water particles of nanometer order and micron order to the container 91 by applying the 8kV between the emitter electrode 20 and the opposed electrode 30. The generation amount of the mist of the charged minute water particles was 2g per an hour. A curve X of FIG.5 shows a weight change ratio of a stick of the wilted celery which was placed in the container 91 for three days. The weight change ratio of a stick of the wilted celery increases to the 102%. And the freshness of the celery is kept. Meanwhile, the curve Y of the FIG.5 shows an experience of using the above mentioned food container 90 without driving the electrostatically atomizing device. In this case, the weight change ratio of the celery three days after decrease to 89%, and the celery becomes more wilted and loses the freshness.

In addition, the experimentation of a decomposition of the agrichemicals from the leafy vegetables was conducted in the above mentioned food container. In this experience, the petri dish storing the fenitrothion (MEP 1 ppm, 0.1 ml) as one example of the agrichemicals was placed at the container 91 which was 30liter volume. The inside of the container 91 was illuminated by the blue LEDs as the dummy sunlight. The electrostatically atomizing device M discharged the mists of the charged minute water particles of nanometer order and micron order to the container 91 by applying 8kV between the emitter electrode 20 and the opposed electrode 30. The generation amount of the mist of the charged minute water particles was 2g per an hour. As a result, the removal ratio of the agrichemicals after 24 hours was 44%. Consequently, it became apparent that the mist of the charged minute water particles had a good removal effect of the agrichemicals. Besides an amount of the radicals generated by the electrostatically atomizing device is 12µM/L.

FIG.6 shows another embodiment of the electrostatically atomizing device in accordance with the present invention. In this embodiment, the electrostatically atomizing device is provided with a piston 44 which is used as the pressurizing means. For this reason, the other configurations and operations are similar to the above embodiment, and duplicate explanations are omitted. The piston 44 is arranged at the pressuring tank 40 which is formed at a rear end of the liquid carrier 10. The piston 44 is driven by an actuator 46, and applies the pressure to the water which is supplied to the liquid carrier 10 from the pressuring tank 40. The actuator 46 is controlled by the pressure regulating means 72, generates the pressure which is selected by the pressure setting means 80, and applies the pressure to the Taylor cone TC which is formed at the tip of the emitter electrode 20. In this embodiment, the pressuring tank 40 is defined as the liquid supplying means and the piston is defined as the pressurizing means.

## Claims

1. An electrostatically atomizing device comprising:
a liquid carrier (10) formed at its one end with an emitter electrode (20);
an opposed electrode (30) disposed in an opposed relation to said emitter electrode (20);
a liquid supplying means for supplying a liquid to said liquid carrier (10);
a high voltage source (60) configured to apply a high voltage between said emitter electrode (20) and said opposed electrode (30) to electrically-charge the liquid fed to a tip of said emitter electrode (20) for discharging a mist of charged minute particles from the tip of said emitter electrode (20);
wherein
said liquid carrier (10) is of a tubular configuration composed of a main tube and a capillary tube which extends from said main tube and which defines said emitter electrode (20), said main tube having an inside diameter sufficiently larger than that of said capillary tube so as to cause no capillary action;
a pressurizing tank (40) configured to apply a water head pressure of the stored liquid to the liquid at the tip of said capillary tube,
wherein
the electrostatically atomizing device further including:
a replenishing tank (50) coupled to said pressurizing tank (40); and
a level sensor (42) configured to detect a liquid level of the liquid in said pressurizing tank (40); and
a replenishing means (52) configured to add the liquid from said replenishing tank (50) to said pressurizing tank (40) in order to keep the liquid level detected by said level sensor (42) at a constant level,
**characterized in that**
said liquid supplying means includes a pressurizing means configured to apply a pressure to the liquid on said emitter electrode (20) for discharging the mist of the charged minute particles of a size in a wide range between a nanometer order of 3 nm to 100 nm and a micron order of 0.1 µm to 10 µm from the tip of said emitter electrode (20), and
said main tube being provided at its rear end with a pressurizing tank (40) which defines said liquid supplying means such that the liquid stored in said pressurising tank (40) applies the pressure to the liquid at the tip of said capillary tube, and **in that** the device comprises a pressure setting means (80) adapted to configure the water head pressure to form the ball of the water at the tip of the emitter electrode (20) by the surface tension, and **in that** the device comprises a controller (70) configured to control the high voltage value applied by the high voltage source (60) such that the Taylor cone (TC) is formed from the water ball to generate the mist of the charged minute water particles of nanometer order from a tip of the Taylor cone (TC) and which generates the mist of the charged minute water particles of micron order from some parts other than the tip of the Taylor cone (TC).

2. An electrostatically atomizing device as set forth in claim 1, wherein
said mist of the charged minute particles of the size in the micron order exhibits a particle size distribution having a peak at 0.5 µm to 1.5 µm.

3. An electrostatically atomizing device as set forth in claim 1, wherein
said mist of the charged minute particles of the size in the nanometer order exhibits a particle size distribution having a peak at 3 nm to 50 nm, and said mist of the charged minute particles of the size in the micron order exhibits a particle size distribution having a peak at 0.5 µm to 1.5 µm.

4. An electrostatically atomizing device as set forth in claim 1, wherein the controller (70) comprises:
a pressure regulating means (72) is provided to regulate the pressure applied to the liquid by said pressurizing means.

5. An electrostatically atomizing device as set forth in claim 4, wherein the pressure regulating means (72) is configured to adjust a particle size distribution of particles of nanometer order and micron order by varying the pressure.

6. An electrostatically atomizing device as set forth in claim 1, wherein
said liquid carrier (10) includes a filter (12) configured to entrap mineral components contained in the liquid.

7. A food container provided with the electrostatically atomizing device as defined in any one of claims 1 to 6.

8. A method of electrostatically atomizing liquid in an electrostatically atomizing device comprising a liquid carrier (10) formed at its one end with an emitter electrode (20), an opposed electrode (30) disposed in an opposed relation to said emitter electrode (20), a liquid supplying means, a high voltage source (60), wherein said liquid supplying means includes a pressurizing means and said liquid carrier (10) is of a tubular configuration composed of a main tube and a capillary tube which extends from said main tube and which defines said emitter electrode (20), said main tube having an inside diameter sufficiently larger than that of said capillary tube so as to cause no capillary action, said main tube being provided at its rear end with a pressurizing tank (40) which defines said liquid supplying means, a replenishing tank (50), a level sensor (42) and a replenishing means (52), the method comprising:
supplying a liquid to the liquid carrier (10);
applying a high voltage between said emitter electrode (20) and said opposed electrode (30) to electrically-charge the liquid fed to a tip of said emitter electrode (20) for discharging a mist of charged minute particles from the tip of said emitter electrode (20);
applying a water head pressure of the stored liquid to the liquid at the tip of said capillary tube;
detecting a liquid level of the liquid in said pressurizing tank (40);
adding the liquid from said replenishing tank (50) to said pressurizing tank (40) in order to keep the liquid level detected by said level sensor (42) at a constant level,
**characterized by**
applying a pressure to the liquid on said emitter electrode (20) for discharging the mist of the charged minute particles of a size in a wide range between a nanometer order of 3 nm to 100 nm and a micron order of 0.1 µm to 10 µm from the tip of said emitter electrode (20);
controlling the water head pressure to form a ball of the water at the tip of the emitter electrode (20) by the surface tension, and in that
applying high voltage to the ball of the water so as to form a Taylor cone (TC) which generates the mist of the charged minute water particles of nanometer order from a tip of the Taylor cone (TC) and which generates the mist of the charged minute water particles of micron order from some parts other than the tip of the Taylor cone (TC).

9. The method of claim 8, further comprising:
adjusting a particle size distribution of particles of nanometer order and micron order by varying the pressure.

## Patentansprüche

1. Elektrostatischer Zerstäuber mit:
einem Flüssigkeitsbeförderer (10), der an seinem einen Ende mit einer Emitterelektrode (20) gebildet ist;
eine Gegenelektrode (30), die in einer gegenüberliegenden Beziehung zu der Emitterelektrode (20) angeordnet ist;
Flüssigkeitsbereitstellungsmittel, um eine Flüssigkeit für den Flüssigkeitsbeförderer (10) bereitzustellen;
einer Hochspannungsquelle (60), die ausgebildet ist, um eine Hochspannung zwischen der Emitterelektrode (20) und der Gegenelektrode (30) anzulegen, um die Flüssigkeit,
die an einer Spitze der Emitterelektrode (20) befördert wird, elektrostatisch aufzuladen, damit sie in Form eines Nebels von geladenen Partikeln von der Spitze der Emitterelektrode (20) freigesetzt wird; wobei
der Flüssigkeitsbeförderer (10) eine tubulare Gestalt aufweist, mit einer Hauptröhre und einer Kapillarröhre, die sich von der Hauptröhre aus erstreckt und die Emitterelektrode (20) definiert, wobei die Hauptröhre einen inneren Durchmesser aufweist, der ausreichend größer ist als die Kapillarröhre, um keine Kapillarwirkung zu erzeugen;
einem Drucktank (40), der ausgebildet ist, um einen Wasserkopfdruck auf die gespeicherte Flüssigkeit hin zu der Flüssigkeit an der Spitze der Kapillarröhre auszuüben, wobei
der elektrostatische Zerstäuber weiter Folgendes umfasst
einen Vorratsbehälter (50), der an dem Drucktank (40) koppelt; und
einen Niveausensor (42), der ausgebildet ist, um ein Flüssigkeitsniveau der Flüssigkeit in dem Drucktank (40) festzustellen; und
eine Nachfülleinheit (52), die ausgebildet ist, um Flüssigkeit von dem Vorratstank (50) in den Drucktank (40) hinzuzufügen, um ein konstantes Flüssigkeitsniveau, das durch den Niveausensor (42) festgestellt wird, auf einem konstanten Niveau zu halten, **dadurch gekennzeichnet, dass**
die Flüssigkeitsbereitstellungsmittel Druckmittel umfassen, die ausgebildet sind, um einen Druck auf die Flüssigkeit an der Emitterelektrode (20) anzuwenden, um den Nebel von geladenen Partikeln zu zerstäuben, wobei die geladenen Partikel eine Größe in einem Bereich zwischen einer Nanometerordnung von 3 nm bis 100 nm und eine Mikrometerordnung von 0,1 µm bis 10 µm, die von der Spitze der Emitterelektrode zerstäubt werden, aufweisen, und
die Hauptröhre an ihrem hinteren Ende mit einem Drucktank (40) ausgebildet ist, der die Flüssigkeitsbereitstellungsmittel definiert, so dass die Flüssigkeit, die in dem Drucktank (40) gespeichert ist, den Druck auf die Flüssigkeit an der Spitze der Kapillarröhre anwendet, und dass
der Zerstäuber ein Druckeinstellmittel (80) umfasst, welches ausgebildet ist, den Wasserkopfdruck derart zu bilden, dass ein Ball von Wasser sich an der Spitze der Emitterelektrode (20) in Folge der Oberflächenspannung herausbildet, und dass der Zerstäuber eine Steuereinheit (70) umfasst, die ausgebildet ist, um die Hochspannung, von der Hochspannungsquelle (60) derart anzuwenden, dass sich ein Taylor Cone aus dem Wasserball bildet, um den Nebel der geladenen Partikel mit der Nanometerordnung von der Spitze des Taylor Cones (TC) zu erzeugen, und um den Nebel von geladenen Partikeln der Mikrometerordnung von den anderen Teilen des Taylor Cones (TC) zu erzeugen.

2. Elektrostatischer Zerstäuber nach Anspruch 1, wobei der Nebel von geladenen Partikeln der Mikrometerordnung eine Teilchengrößenverteilung aufweist, die ein Maximum bei 0,5 µm bis 1,5 µm hat.

3. Elektrostatischer Zerstäuber nach Anspruch 1, wobei
der Nebel von geladenen Partikeln der Nanometerordnung eine Teilchengrößenverteilung aufweist, die ein Maximum bei 3 nm bis 50 nm hat, und wobei der Nebel von geladenen Partikeln der Mikrometerordnung eine Teilchengrößenverteilung aufweist, die ein Maximum hat bei 0,5 µm bis 1,5 µm.

4. Elektrostatischer Zerstäuber nach Anspruch 1, wobei die Steuereinheit (70) Folgendes umfasst:
ein Druckregulierungsmittel (72), welches bereitgestellt ist, um den Druck, der auf die Flüssigkeit in dem Druckausübungsmittel wirkt, zu regulieren.

5. Elektrostatischer Zerstäuber nach Anspruch 4, wobei das Druckregulierungsmittel (72) ausgebildet ist, um die Teilchengrößenverteilung der Partikel mit der Nanometerordnung und der Mikrometerordnung durch eine Änderung des Druckes zu verändern.

6. Elektrostatischer Zerstäuber nach Anspruch 1, wobei der Flüssigkeitsbeförderer (10) einen Filter (12) umfasst, der ausgebildet ist, um mineralische Anteile, die in der Flüssigkeit enthalten sind, herauszufiltern.

7. Lebensmittelbehälter mit einem elektrostatischen Zerstäuber nach einem der Ansprüche 1-6.

8. Verfahren zum elektrostatischen Zerstäuben einer Flüssigkeit in einem elektrostatischen Zerstäuber mit einem Flüssigkeitsbeförderer (10), der an seinen einen Ende mit einer Emitterelektrode (20), einer Gegenelektrode (30), die an einer gegenüberliegenden Seite in Bezug auf die Emitterelektrode (20) angeordnet ist, einem Flüssigkeitsbereitstellungsmittel, einer Hochspannungsquelle (60), wobei das Flüssigkeitsbereitstellungsmittel ein Druckerzeugungsmittel umfasst und der Flüssigkeitsbeförderer (10) eine tubulare Form aufweist, die durch eine Hauptröhre und durch eine Kapillarröhre gebildet ist, wobei sich die Kapillarröhre von der Hauptröhre aus erstreckt und die Emitterelektrode (20) definiert, wobei die Hauptröhre einen inneren Durchmesser aufweist, der ausreichend größer ist als jener der Kapillarröhre, so dass keine Kapillarwirkung erzeugt wird, wobei die Hauptröhre an ihrem hinteren Ende mit einem Drucktank (40) ausgebildet ist, welcher das Flüssigkeitsbereitstellungsmittel definiert, einem Vorratstank (50), einem Höhenstandsensor (42) und einem Nachfüllmittel (52) umfasst, wobei das Verfahren Folgendes umfasst:
Bereitstellen einer Flüssigkeit für das Flüssigkeitsbefördermittel (10);
Anwenden einer Hochspannung zwischen der Emitterelektrode (20) und der Gegenelektrode (30), um die Flüssigkeit, die der Spitze der Emitterelektrode (20) zugeführt wird, elektrostatisch aufzuladen, um einen Nebel von geladenen Partikeln von der Spitze der Emitterelektrode (20) abzugeben;
Anwenden eines Wasserkopfdruckes auf die gespeicherte Flüssigkeit hin zu der Flüssigkeit an der Spitze der Kapillarröhre;
Feststellen eines Flüssigkeitshöhenstandes der Flüssigkeit in den Drucktank (40);
Hinzufügen der Flüssigkeit von dem Vorratstank (50) zu dem Drucktank (40), um das Flüssigkeitsniveau, welches durch den Höhenstandsensor (42) festgestellt wurde, auf einem konstante Niveau zu halten,
**gekennzeichnet durch**
Anwenden eines Druckes auf die Flüssigkeit an der Emitterelektrode (20), um den Nebel von geladenen Partikeln derart zu erzeugen, dass eine Größe in einem weiten Bereich zwischen einer Nanometerordnung von 3 nm bis 100 nm und einer Mikrometerordnung von 0, 1 µm bis zu 10 µm von der Spitze der Emitterelektrode (20) erreicht wird;
Steuern des Wasserkopfdruckes um einen Ball von Wasser an der Spitze der Emitterelektrode (20) in Folge der Oberflächenspannung zu erzeugen, und indem eine Hochspannung auf den Wasserball angewandt wird, um einen Taylor Cone (TC) zu bilden, der den Nebel von geladenen Partikeln der Nanometerordnung von der Spitze des Taylor Cones (TC) erzeugt und welcher den Nebel von geladenen Partikeln der Mikrometerordnung von den anderen Teilen des Taylor Cones (TC) erzeugt.

9. Verfahren nach Anspruch 8, welches weiter Folgendes umfasst:
Einstellen einer Teilchengrößenverteilung der Nanometerordnung und der Mikrometerordnug durch ein Ändern des Druckes.

## Revendications

1. Dispositif d'atomisation électrostatique comprenant :
un support de liquide (10) comportant à son extrémité une électrode émettrice (20) ;
une électrode opposée (30) disposée dans une relation de vis-à-vis avec ladite électrode émettrice (20) ;
des moyens de fourniture de liquide pour fournir un liquide audit support de liquide (10) ;
une source haute tension (60) configurée pour appliquer une haute tension entre ladite électrode émettrice (20) et ladite électrode opposée (30) pour charger électriquement le liquide fourni à une extrémité de ladite électrode émettrice (20) pour décharger un brouillard de minuscules particules chargées de l'extrémité de ladite électrode émettrice (20) ;
dans lequel
ledit support de liquide (10) a une configuration tubulaire composée d'un tube principal et d'un tube capillaire qui s'étend dudit tube principal et qui définit ladite électrode émettrice (20), ledit tube principal ayant un diamètre intérieur suffisamment plus grand que celui dudit tube capillaire pour ne provoquer aucune action de capillarité ;
un réservoir de pressurisation (40) configuré pour appliquer une pression de charge d'eau du liquide stocké au liquide à l'extrémité dudit tube capillaire,
dans lequel
le dispositif d'atomisation électrostatique comprend en outre :
un réservoir de remplissage (50) accouplé audit réservoir de pressurisation (40) ; et
un capteur de niveau (42) configuré pour détecter un niveau de liquide du liquide dans ledit réservoir de pressurisation (40) ; et
des moyens de remplissage (52) configurés pour ajouter le liquide dudit réservoir de remplissage (50) dans ledit réservoir de pressurisation (40) afin de maintenir le niveau de liquide détecté par ledit capteur de niveau (42) à un niveau constant,
**caractérisé en ce que**
lesdits moyens de fourniture de liquide comprennent des moyens de pressurisation configurés pour appliquer une pression au liquide sur ladite électrode émettrice (20) pour décharger le brouillard de minuscules particules chargées d'une taille dans une large plage de l'ordre du nanomètre de 3 nm à 100 nm à l'ordre du micron de 0,1 µm à 10 µm de l'extrémité de ladite électrode émettrice (20), et
ledit tube principal est pourvu, à son extrémité arrière, d'un réservoir de pressurisation (40) qui définit lesdits moyens de fourniture de liquide de sorte que le liquide stocké dans ledit réservoir de pressurisation (40) applique la pression au liquide à l'extrémité dudit tube capillaire, et **en ce que** le dispositif comprend :
des moyens de réglage de pression (80) conçus pour configurer la pression de charge d'eau pour former la bille de l'eau à l'extrémité de l'électrode émettrice (20) par la tension superficielle, et **en ce que** le dispositif comprend :
un contrôleur (70) configuré pour commander la valeur de haute tension appliquée par la source haute tension (60) de sorte que le cône de Taylor (TC) soit formé à partir de la bille d'eau pour générer le brouillard de minuscules particules d'eau chargées de l'ordre du nanomètre à partir d'une extrémité du cône de Taylor (TC) et qui génère le brouillard de minuscules particules d'eau chargées de l'ordre du micron à partir de certaines parties autres que l'extrémité du cône de Taylor (TC).

2. Dispositif d'atomisation électrostatique selon la revendication 1, dans lequel
ledit brouillard de minuscules particules chargées de la taille de l'ordre du micron présente une distribution de taille de particule comportant un pic de 0,5 µm à 1,5 µm.

3. Dispositif d'atomisation électrostatique selon la revendication 1, dans lequel
ledit brouillard de minuscules particules chargées de la taille de l'ordre du nanomètre présente une distribution de taille de particule ayant un pic de 3 nm à 50 nm, et ledit brouillard de minuscules particules chargées de la taille de l'ordre du micron présente une distribution de taille de particule ayant un pic de 0,5 µm à 1,5 µm.

4. Dispositif d'atomisation électrostatique selon la revendication 1, dans lequel le contrôleur (70) comprend :
des moyens de régulation de pression (72) prévus pour réguler la pression appliquée au liquide par lesdits moyens de pressurisation.

5. Dispositif d'atomisation électrostatique selon la revendication 4, dans lequel les moyens de régulation de pression (72) sont configurés pour ajuster une distribution de taille de particule des particules de l'ordre du nanomètre et de l'ordre du micron en modifiant la pression.

6. Dispositif d'atomisation électrostatique selon la revendication 1, dans lequel
ledit support de liquide (10) comprend un filtre (12) configuré pour piéger des composants minéraux contenus dans le liquide.

7. Récipient de nourriture pourvu du dispositif d'atomisation électrostatique selon l'une quelconque des revendications 1 à 6.

8. Procédé d'atomisation électrostatique de liquide dans un dispositif d'atomisation électrostatique comprenant un support de liquide (10) comportant à son extrémité une électrode émettrice (20), une électrode opposée (30) qui est disposée dans une relation de vis-à-vis avec ladite électrode émettrice (20), des moyens de fourniture de liquide, une source haute tension (60), dans lequel lesdits moyens de fourniture de liquide comprennent des moyens de pressurisation et ledit support de liquide (10) a une configuration tubulaire composée d'un tube principal et d'un tube capillaire qui s'étend dudit tube principal et qui définit ladite électrode émettrice (20), ledit tube principal ayant un diamètre intérieur suffisamment plus grand que celui dudit tube capillaire pour ne provoquer aucune action de capillarité, ledit tube principal étant pourvu, à son extrémité arrière, d'un réservoir de pressurisation (40) qui définit lesdits moyens de fourniture de liquide, d'un réservoir de remplissage (50), d'un capteur de niveau (42) et de moyens de remplissage (52), le procédé consistant à :
fournir un liquide au support de liquide (10) ;
appliquer une haute tension entre ladite électrode émettrice (20) et ladite électrode opposée (30) pour charger électriquement le liquide appliqué à une extrémité de ladite électrode émettrice (20) pour décharger un brouillard de minuscules particules chargées de l'extrémité de ladite électrode émettrice (20) ;
appliquer une pression de charge d'eau du liquide stocké au liquide à l'extrémité dudit tube capillaire ;
détecter un niveau de liquide du liquide dans ledit réservoir de pressurisation (40) ;
ajouter le liquide dudit réservoir de remplissage (50) audit réservoir de pressurisation (40) afin de maintenir le niveau de liquide détecté par ledit capteur de niveau (42) à un niveau constant,
**caractérisé par** les étapes consistant à :
appliquer une pression au liquide sur ladite électrode émettrice (20) pour décharger le brouillard de minuscules particules chargées d'une taille dans une large plage de l'ordre du nanomètre de 3 nm à 100 nm à l'ordre du micron de 0,1 µm à 10 µm de l'extrémité de ladite électrode émettrice (20) ;
commander la pression de charge d'eau pour former une bille de l'eau à l'extrémité de l'électrode émettrice (20) par la tension superficielle, et par l'étape consistant à :
appliquer une haute tension à la bille de l'eau de manière à former un cône de Taylor (TC) qui génère le brouillard de minuscules particules d'eau chargées de l'ordre du nanomètre à partir d'une extrémité du cône de Taylor (TC) et qui génère le brouillard de minuscules particules d'eau chargées de l'ordre du micron à partir de certaines parties autres que l'extrémité du cône de Taylor (TC).

9. Procédé selon la revendication 8, consistant en outre à :
ajuster une distribution de taille de particule des particules de l'ordre du nanomètre et de l'ordre du micron en modifiant la pression.
